(19) Europäisches Patentamt
European Patent Office
Office européen des brevets

(11) Veröffentlichungsnummer : **0 339 411 B1**

(12) # EUROPÄISCHE PATENTSCHRIFT

(45) Veröffentlichungstag der Patentschrift :
**12.08.92 Patentblatt 92/33**

(51) Int. Cl.[5] : **C07D 241/44**

(21) Anmeldenummer : **89106784.5**

(22) Anmeldetag : **15.04.89**

(54) **Verfahren zur Herstellung von Chinoxalonen.**

(30) Priorität : **26.04.88 DE 3813977**
**19.01.89 DE 3901406**

(43) Veröffentlichungstag der Anmeldung :
**02.11.89 Patentblatt 89/44**

(45) Bekanntmachung des Hinweises auf die Patenterteilung :
**12.08.92 Patentblatt 92/33**

(84) Benannte Vertragsstaaten :
**BE CH DE FR GB IT LI NL**

(56) Entgegenhaltungen :
**EP-A- 0 295 815**
**US-A- 4 636 562**

(56) Entgegenhaltungen :
**JOURNAL OF THE ROYAL NETHERLANDS CHEMICAL SOCIETY Band 99, Nr. 4, April 1980, Seiten 115-117; B. TE NIJENHUIS et al.:"Photochemcial hydroxylation of 7,8-dimethylalloxazine (lumichrome). Structure elucidation of the irradiation product" Seite 117,Spalte 1, Zeilen 56-68**

(73) Patentinhaber : **HOECHST AKTIENGESELLSCHAFT**
**Postfach 80 03 20**
**W-6230 Frankfurt am Main 80 (DE)**

(72) Erfinder : **Daub, Wolfgang, Dr.**
**Münsterer Strasse 37a**
**W-6233 Kelkheim (Taunus) (DE)**
Erfinder : **Papenfuhs, Theodor, Dr.**
**Heinrich-Bleicher-Strasse 40**
**W-6000 Frankfurt am Main 50 (DE)**
Erfinder : **Planker, Siegfried, Dr.**
**Kastanienweg 17**
**W-6240 Königstein/Taunus (DE)**

EP 0 339 411 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Gegenstand der Erfindung ist ein gegenüber den bekannten Verfahren verbessertes Verfahren zur Herstellung von Chinoxalonen durch katalytische Reduktion (Hydrierung) von Chinoxalon-N-oxiden. Chinoxalone stellen u.a. wertvolle Vorprodukte zur Herstellung von Pflanzenschutzmitteln dar [DE-OS 30 04 770].

Es ist bekannt, daß Chinoxalon-N-oxid der Formel

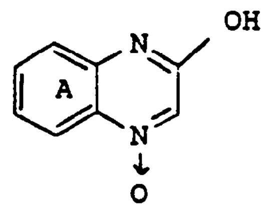

welches im Benzolkern A substituiert sein kann, beispielsweise durch Halogenatome, Halogenalkyl-, Alkyl-, Methoxy- oder Nitrogruppen, prinzipiell unter Anwendung verschiedener Reduktionsmittel zum entsprechenden Chinoxalon der Formel

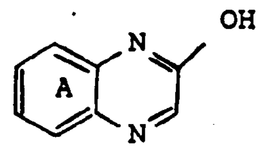

reduziert werden kann. Alle bekannten Verfahren weisen jedoch schwerwiegende Mängel auf, weshalb sie für eine Nutzung im technischen Maßstab wenig geeignet sind.

Auf die wichtigsten bekannten Verfahren sei nachstehend eingegangen.

Von G. Sakata, K. Makino and K. Morimoto werden in HETEROCYCLES 23, 143 (1985) zwei verschiedene Verfahren zur Reduktion einiger Chinoxalon-N-oxide beschrieben (siehe hierzu auch J 57-188 575 A und J 57-197 270 A). Die besten Ergebnisse hinsichtlich Ausbeute und Produktqualität werden hierbei durch Anwendung des kostspieligen Natriumborhydrids als Reduktionsmittel erzielt. Das dort beschriebene Verfahren unter Verwendung des genannten Reduktionsmittels ist wegen der sehr aufwendigen Handhabung des genannten Reduktionsmittels für eine Durchführung im technischen Maßstab wenig befriedigend. Ebenfalls sehr gute Ausbeuten wurden von den genannten Autoren bei der Reduktion von Chinoxalon-N-oxiden mit wäßrigen Sulfitlaugen erzielt. Nachteilig hierbei ist, daß nicht unbeträchtliche Mengen an entsprechenden 2,3-Dihydroxychinoxalinen entstehen, die bei einer Weiterverarbeitung der erhaltenen Chinoxalone stören, von diesen aber nicht in befriedigender Weise abgetrennt werden können. Hinzukommt, daß bei diesem bekannten Verfahren eine mit Sulfit bzw. Sulfat belastete Ablauge entsteht, die nicht regeneriert werden kann und bei der Entsorgung ernste Probleme mit sich bringt. Aus den genannten Gründen ist auch das letztgenannte Verfahren für eine industrielle Verwertung wenig geeignet.

Von Ishikura, Tsukasa, Ageo und Saitama wird in der DE-OS 3 419 471 ein Verfahren zur Herstellung von Chinoxalonen durch Reduktion von Chinoxalon-N-oxiden mit Hydrazin in Gegenwart von Raney-Nickel beschrieben. Hierbei werden zwar ebenfalls sehr gute Ausbeuten und Produktqualitäten erzielt, jedoch ist die Anwendung von Hydrazin bei einem technischen Verfahren, insbesondere aufgrund toxikologischer Probleme, von großem Nachteil.

Von verschiedenen anderen Autoren wird für die Reduktion von Chinoxalon-N-oxiden die Anwendung einer Reihe weiterer Reduktionsmittel beschrieben, wie beispielsweise Natriumdithionit, Natriumarsenit, Ammoniumsulfid oder Metalle, wie Zink, Eisen oder Zinn, in Gegenwart von Säuren. Diese sind jedoch in allen Fällen weniger selektiv, liefern geringere Ausbeuten und führen zur Bildung von Nebenprodukten, wie beispielsweise 2,3-Dihydroxychinoxaline und/oder 3,4-Dihydrochinoxalone. Das ebenfalls als Reduktionsmittel beschriebene Triphenylphosphin ist kostspielig und erfordert hohe Reaktionstemperaturen. Entsprechende Zusammenstellungen und zugehörige Literaturzitate finden sich in den vorstehend genannten Literaturstellen sowie in der US-PS 4 636 562.

Unbefriedigend ist auch die katalytische Hydrierung von Chinoxalon-N-oxiden unter Verwendung von Palladium-Katalysatoren. Nach G. Sakata, K. Makino und K. Morimoto [HETEROCYCLES 23, 143 (1985)] ist dieses Verfahren nicht selektiv. Als wichtigste Nebenprodukte werden hierbei in erheblichem Maße 3,4-Dihydrochinoxalone gebildet, die in umständlicher Weise zu den gewünschten Chinoxalonen zurückoxidiert werden müssen. Bei der Reduktion von am Benzolkern halogenierten Chinoxalon-N-oxiden, beispielsweise von 6-Chlorochinoxalon-N-oxid, kommt es außerdem zu einer teilweisen Enthalogenierung. Entsprechende Nebenprodukte würden bei der Weiterverarbeitung der erhaltenen Chinoxalone stören.

In der US-PS 4 636 562 wird ein Verfahren zur katalytischen Hydrierung von Chinoxalon-N-oxiden in Gegenwart der Übergangsmetalle Platin, Rhodium, Nickel, Ruthenium, Kobalt und Kupfer in Wäßrig-alkalischer Lösung beschrieben. Das dort aufgeführte Ausführungsbeispiel liefert unter Verwendung von Raney-Nickel als Katalysator das 6-Chlorchinoxalon nur in einer unbefriedigenden Ausbeute, obwohl der Katalysator mit einem gegenüber der Ausgangsverbindung (6-Chlorchinoxalon-N-oxid) beträchtlichen Überschuß eingesetzt wurde. Der dort angegebene Schmelzpunkt von > 250°C und die dort angegebene Farbe der genannten 6-Chlor-Verbindung unterscheiden sich beträchtlich von den in den weiter oben zitierten Literaturstellen und in den Beispielen der vorliegenden Erfindung angegebenen Daten. So wird der Schmelzpunkt in den weiter oben angegebenen Literaturstellen und in den vorliegenden Beispielen 1 und 2 mit > 300°C angegeben. Während in der zitierten US-PS die inredestehende Verbindung als lavendelfarbig bezeichnet wird, wird diese in HETEROCYCLES 23, 143 (1985) als farblos und in Beispielen der vorliegenden Erfindung als hellbeige (bei guter Produktqualität) bzw. als braunrosa bis lila (bei Gegenwart erheblicher Mengen von 2,3-Dihydroxychinoxalinen als Nebenprodukt) bezeichnet. Diese Unterschiede im Schmelzpunkt und in der Farbe bei Fehlen weiterer Reinheitskriterien zwingen zu dem Schluß, daß das gemäß der zitierten US-PS erhaltene Produkt in ungenügender Qualität anfällt und durch Nebenprodukte verunreinigt ist (vgl. hierzu das weiter unten angegebene Vergleichsbeispiel 2). Die Anwendung weiterer Katalysatoren aus der dort angegebenen Reihe von Übergangsmetallen wird nicht durch Ausführungsbeispiele belegt.

Es muß somit insgesamt aus den vorliegenden Daten der vorstehend zitierten Literaturstellen der Schluß gezogen werden, daß nach dem Stand der Technik die katalytische Hydrierung von im Benzolkern A ggfs. substituierten Chinoxalon-N-oxiden mit Wasserstoff in Gegenwart der vorstehend genannten Übergangsmetalle als Hydrierkatalysatoren nicht mit ausreichender Selektivität erfolgt und allgemein zu einer unbefriedigenden Produktqualität führt.

Es wurde nun überraschenderweise gefunden, daß man Chinoxalone der allgemeinen Formel (I)

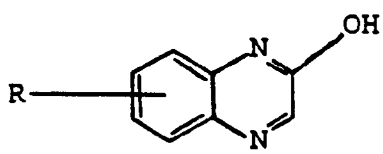

(I)

in welcher R ein Wasserstoffatom oder 1 bis 4 gleiche oder verschiedene Substituenten aus der Reihe Halogenatome, vorzugsweise Fluor- oder Chloratome, Alkyl-$C_1$-$C_4$-, Cycloalkyl-$C_3$-$C_6$-, Alkoxy-$C_1$-$C_4$, Trifluormethyl-, Tetrafluorethoxy-, Hydroxyl-, Carboxyl-, Amino-, Monoalkyl-$C_1$-$C_4$-amino- oder Dialkyl-$C_1$-$C_4$-aminogruppen bedeuten, auf einfache Weise in hohen Ausbeuten und in hervorragender Selektivität herstellen kann, indem man Chinoxalon-N-oxide der allgemeinen Formel (II)

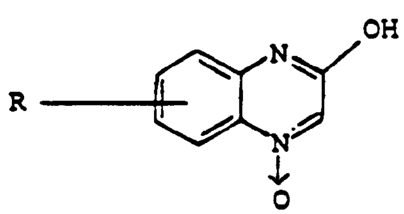

(II)

in welcher R die vorstehend genannten Bedeutungen hat und außerdem auch eine Nitrogruppe sein kann, die während des Verfahrens unter den Verfahrensbedingungen zu einer Aminogruppe reduziert wird, in wäßrig-alkalischer Lösung in Gegenwart von etwa 0,001 bis etwa 0,5 Molprozent, vorzugsweise von etwa 0,01 bis etwa 0,05 Molprozent eines Platin-Schalenkatalysators, bezogen auf eingesetztes Chinoxalon-N-oxid, bei Temperaturen von etwa 20 bis etwa 120°C, vorzugsweise etwa 60 bis etwa 100°C, bei einem Wasserstoffdruck von etwa 1 bis etwa 100 bar, vorzugsweise von etwa 5 bis etwa 20 bar, in Gegenwart von etwa 1 bis etwa 25 Gewichtsprozent, vorzugsweise von etwa 2 bis etwa 5 Gewichtsprozent, eines Alkalimetallhydroxids, vorzugsweise Natrium- oder Kaliumhydroxid, bezogen auf die Lösung, hydriert.

In Abhängigkeit von Art und Zahl der Substituenten R am Benzolkern der Chinoxalon-N-oxide kann es vorteilhaft sein, in Gegenwart von Polyethylenglykolen, insbesondere solchen der allgemeinen Formel III

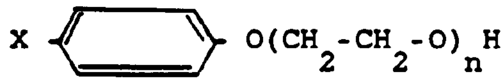

(III)

in welcher X eine Alkyl-$C_4$-$C_{12}$-Gruppe und n eine Zahl von etwa 5 bis etwa 20 bedeuten, zu hydrieren. Die genannten Hilfsmittel werden in einer Menge von etwa 0,5 bis etwa 10 Gewichtsprozent, vorzugsweise etwa 2 bis etwa 7 Gewichtsprozent, bezogen auf eingesetztes Chinoxalon-N-oxid (Formel (II)), eingesetzt.

An Ausgangsverbindungen der genannten allgemeinen Formel II seien beispielsweise folgende aufgeführt:
Chinoxalon-N-oxid, 5-, 6- und 7-Chlorochinoxalon-4-oxid, 6- und 7-Fluorchinoxalon-4-oxid, 6,7-Dichlorochinoxalon-4-oxid, 6,8-Dichlorchinoxalon-4-oxid, 6,7-Difluorchinoxalon-4-oxid, 5,6,7,8-Tetrafluorchinoxalon-4-oxid, 5-, 6- und 7-Methylchinoxalon-4-oxid, 6,7-Dimethylchinoxalon-N-oxid, 6- und 7-Methoxychinoxalon-4-oxid, 6-Ethoxychinoxalon-4-oxid, 6-Butoxychinoxalon-4-oxid, 6-Chlor-7-methoxychinoxalon-4-oxid, 6- und 7-Trifluormethylchinoxalon-4-oxid, 6- und 7-Hydroxychinoxalon-4-oxid, Chinoxalon-4-oxid-6-carbonsäure.

Zum erfindungsgemäßen Verfahren sei im einzelnen noch folgendes ausgeführt:
In der Regel ist es zweckmäßig, die Ausgangsverbindung der genannten allgemeinen Formel II in der wäßrig-alkalischen Lösung in einer Konzentration von etwa 2 bis etwa 20 Gewichtsprozent, vorzugsweise von etwa 3 bis etwa 15 Gewichtsprozent, einzusetzen. Die katalytische Hydrierung nimmt, in Abhängigkeit der Reaktionsparameter, etwa 30 Minuten bis etwa 5 Stunden, in der Regel etwa 1 bis etwa 3 Stunden in Anspruch.

Als besonderer Vorteil des erfindungsgemäßen Verfahrens sei hervorgehoben, daß die Bildung von Nebenprodukten, wie sie durch die nachstehenden Formeln IV und V repräsentiert werden, sowie im Falle von R = Chlor eine Entchlorierung nur in unbedeutendem Maße (üblicherweise < 1%) beobachtet wird.

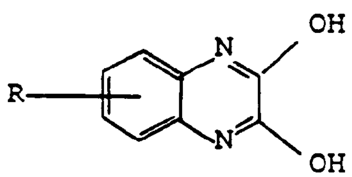

(IV)

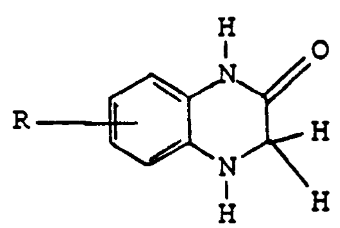

(V)

Nach dem erfindungsgemäßen Verfahren gelingt es somit, die Verbindungen der genannten allgemeinen Formel I in einfacher Weise kostengünstig, praktisch ohne ökologische Nachteile, in sehr guten Ausbeuten und in hoher Selektivität herzustellen, weshalb sie die an eine Weiterverarbeitung zu stellenden Bedingungen erfüllen.

Der eingesetzte Katalysator kann wiederholt eingesetzt werden.

Ein zusätzlicher Vorteil des erfindungsgemäßen Verfahrens ist, daß im Falle der Darstellung von Chinoxalon-N-oxiden der genannten allgemeinen Formel II durch Cyclisierung von ortho-Nitroacetoacetylaniliden auf eine Zwischenisolierung der Chinoxalon-N-oxide verzichtet werden kann und die dabei erhaltene alkalische Reaktionsmischung, gegebenenfalls nach Verdünnen und/oder einer Klärfiltration, direkt für die katalytische Hydrierung eingesetzt werden kann.

Die Mengen an Platin-Schalenkatalysator und Alkalimetallhydroxid sowie Temperatur und Wasserstoffdruck können zwar über die jeweilige Obergrenze der angegebenen Bereiche hinausgehen. Dies bringt aber praktisch keine zusätzliche Verbesserung im Verfahren.

**Nähere Erläuterung des Begriffs Schalenkatalysator:**

Als Schalenkatalysatoren werden solche Hydrierkatalysatoren bezeichnet, bei denen das Edelmetall z.B. Platin, in feinster Form überwiegend auf der Oberfläche eines Trägermaterialteilchens, z.B. Aktivkohle, verteilt ist (s. Abbildung I) im Gegensatz zu als gewöhnlich zu bezeichnenden Edelmetallkatalysatoren, die eine überwiegend einheitliche Verteilung des Edelmetalls auch innerhalb (über Poren etc.) eines Trägermaterialteilchens aufweisen (s. Abbildung II).

Querschnitt der belegten Trägermaterialteilchen bei einem Schalenkatalysator (Abbildung I) und einem gewöhnlichen Trägerkatalysator (Abbildung II):

Abb. I 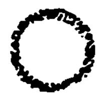

Abb. II 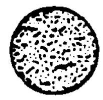

Die Herstellung von Katalysatoren des Typs Schalenkatalysator erfordert spezielle Kenntnisse, die in der Regel nur beim Hersteller des jeweiligen Katalysators vorliegen. Für die nachfolgenden Ausführungsbeispiele wurde ein spezieller, käuflicher Schalenkatalysator der Firma DEGUSSA eingesetzt. Prinzipiell sind jedoch auch andere Katalysatoren einsetzbar, wenn sie entsprechende Strukturmerkmale aufweisen.

Katalysatoren der herkömmlichen Struktur, analog Abbildung II, sind in Übereinstimmung mit der zitierten Literatur und den entsprechenden Vergleichsbeispielen zur vorliegenden Erfindung für das erfindungsgemäße Verfahren nicht geeignet.

Die nachstehenden Beispiele dienen zur Erläuterung des erfindungsgemäßen Verfahrens.

**Beispiel 1**

118 g (0,6 Mol) 6-Chlorchinoxalon-4-oxide (Herstellung analog Beispiel 2) in Gegenwart von (p-Nonylphenyl)-polyglykolether wurden in 900 g ca. 3 %iger Natronlauge gelöst und in Gegenwart von 2 g Platinschalenkatalysator [Typ 2.5 Pt/C, F 101 KY/W mit ca. 50 Gewichtsprozent Wasser (DEGUSSA)] auf 80 - 90°C erhitzt und mit Wasserstoff unter einem Druck von 10 bar 1,5 Stunden in einem Autoklav hydriert. Der Katalysator wurde anschließend über eine Drucknutsche abgetrennt und das Filtrat mit Salzsäure auf pH 6 bis 7 gestellt. Nach Absaugen wurde das beigefarbene Produkt bei 60 bis 80°C im Vakuum getrocknet.

Gewonnen wurden 104,5 g 6-Chlorchinoxalon mit einem Reingehalt von 83 % (HPLC externer Standard; Rest überwiegend Salze) ≙ 86,7 g (0.48 Mol) 6-Chlorchinoxalon, gerechnet 100 % ≙ 80 % der Theorie. Schmelzpunkt 310 bis 315°C, $^{1}$H-NMR in Einklang mit der gewünschten Struktur.

Nebenprodukte nach HPLC: ca. 1 % 3,4-Dihydro-6-chlorchinoxalon; Chinoxalon: in Spuren; 6-Chlor-2,3-dihydroxychinoxalin: nicht nachweisbar; weitere organische Verunreinigungen: höchstens in Spuren.

**Beispiel 2**

256,6 g (1.06 Mol) 4-Chlor-2-nitroacetylacetanilid wurden innerhalb von 1,5 Stunden bei 60°C in 1200 g 16%ige Natronlauge, die 10 g (p-Nonylphenyl)-polyglykolether enthielt, eingetragen. Nach insgesamt 3 Stunden wurde die erhaltene hellbraune Suspension bis zur vollständigen Lösung mit Wasser verdünnt, filtriert und anschließend, wie in Beispiel 1 beschrieben, hydriert.

Gewonnen wurden 174 g 6-Chlorchinoxalon mit einem Reingehalt von 83 %, was einer Ausbeute von 80 % der Theorie entspricht.

Schmelzpunkt: > 300°C, $^{1}$H-NMR in Einklang mit der gewünschten Struktur.

Nebenprodukte nach HPLC: Chinoxalon < 1 %; 6-Chlor-2,3,-dihydroxychinoxalin << 1 %; 6-Chlor-3,4,-dihydrochinoxalon < 1 %; weitere organische Verunreinigungen: höchstens in Spuren.

**Beispiel 3**

48,5 g (0,2 Mol) 4-Methyl-2-nitroacetylacetanilid wurden innerhalb 1 Stunde bei 60°C in 240 g 16 %ige Natronlauge, die 2 g (p-Nonylphenyl)-polyglykolether enthielt, eingetragen. Nach insgesamt 2 Stunden wurde die erhaltene Suspension mit 1 l Wasser verdünnt, filtriert und anschließend 3 Stunden bei 100°C unter einem Wasserstoffdruck von 10 bar hydriert. Die Aufarbeitung erfolgte analog Beispiel 1.

Gewonnen wurden 23,5 g 6-Methylchinoxalon ≙ 73 % der Theorie.
Schmelzpunkt: 268 - 271°C
$^{1}$H-NMR*: 12,34 (s, 1H, OH); 8,15 (s, 1H, $H^3$); 7,57 (d $^4J_{5,7}$=1,8 Hz, 1H, $H^5$); 7,33 (dd, $^3J_{7,8}$=8,4 Hz, $^4J_{5,7}$ = 1,8

Hz, 1H, $H^7$); 7,21 (d, $^3J_{7,8}$=8,4 Hz, 1H, $H^8$); 2,38 (s, 3H, $CH_3$).

**Beispiel 4**

87,0 g (0,3 Mol) 4-(Trifluormethyl)-2-nitroacetylacetanilid wurden analog Beispiel 3 umgesetzt. Die Hydrierung war nach 2 Stunden bei 80 - 90°C beendet.

Gewonnen wurden 48,6 g 6-(Trifluormethyl)-chinoxalon ≙ 75,6 % der Theorie.
Schmelzpunkt: 250 - 252°C
$^1$H-NMR*: 12,7 ($\bar{s}$, 1H, OH); 8,28 (s, 1H, $CH^3$); 8,05 (s, 1H, $CH^5$); 7,86 (d, $^3J_{7,8}$=7,5 Hz, 1H, $CH^7$); 7,47 (d, $^3J_{7,8}$=8,4 Hz, 1H, $CH^8$).

**Beispiel 5**

77,0 g (0,3 Mol) 5-Chlor-2-nitroacetylacetanilid wurden analog Beispiel 3 umgesetzt. Die Hydrierung war nach 3 Stunden bei 80 - 90°C beendet.

Gewonnen wurden 43,3 g 7-Chlorchinoxalon ≙ 79,9 % der Theorie.
Schmelzpunkt: 252 - 254°C.
$^1$H-NMR*: 12,47 (s, 1H, 0H); 8,17 (s, 1H, $H^3$); 7,77 (d, $^3J_{5,6}$=8,4 Hz, 1H, $H^5$); 7,32 (dd, $^3J_{5,6}$=8,4 Hz, $^4J_{6,8}$ = 2,1 Hz, 1H, $H^6$); 7,29 (d, $^4J_{6,8}$=1,8 Hz, 1H, $H^8$).

**Beispiel 6**

25,2 g (0,1 Mol) 4-Methoxy-2-nitroacetylacetanilid wurden analog Beispiel 3 umgesetzt. Die Hydrierung war nach 2 Stunden bei 80 - 90°C beendet.

Gewonnen wurden 12,5 g 6-Methoxychinoxalon ≙ 71,0 % der Theorie.
Schmelzpunkt: 252 - 257°C
$^1$H-NMR*: 12,3 ($\bar{s}$, 1H, OH); 8,17 (s, 1H, $H^3$); 7,25 (m, 3H, $H^{5,7,8}$); 3,83 (s, 3H, $OCH_3$).

**Beispiel 7**

90,4 g (0,4 Mol) 2-Nitroacetylacetanilid wurden analog Beispiel 3 umgesetzt. Die Hydrierung war nach 2 Stunden bei 80 - 90°C beendet.

Gewonnen wurden 44,2 g 2-Chinoxalon ≙ 75,7 % der Theorie.
Das $^1$H-NMR entsprach dem einer handelsüblichen Vergleichssubstanz.
Schmelzpunkt: 259°C.

**Beispiel 8**

47,1 g (0,16 Mol) 4-(Acetoacetylamino)-3-nitrobenzoesäureethylester wurden analog Beispiel 3 umgesetzt (die Esterfunktion wird dabei zur Carbonsäure verseift). Die Hydrierung war nach 2 Stunden bei 80 - 90°C beendet.
Gewonnen wurden 23,5 g 2-Hydroxychinoxalin-6-carbonsäure ≙ 77 % der Theorie.
Schmelzpunkt: Das Produkt schmilzt nicht unter 300°C.
$^1$H-NMR*: 12,4 ($\bar{s}$, 1H, OH); 8,27 (d, $^4J_{5,7}$=1,8 Hz, 1H, $H^5$); 8,24 (s, 1H, $H^3$); 8,08 (dd, $^3J_{7,8}$=8,4 Hz, $^4J_{5,7}$=1,8 Hz, 1H, $H^7$); 7,37 (d, $^3J_{7,8}$=8,7 Hz, 1H, $H^8$); 3,5 ($\bar{s}$, COOH).

* Die $^1$H-NMR-Spektren wurden bei 300 MHz in Dimethylsulfoxid (d6) als Lösungsmittel gewonnen. Chemische Verschiebungen sind in ppm angegeben. Die Abbkürzungen bedeuten:
s: Singulett; $\bar{s}$: verbreitertes Singulett;
d: Dublett; dd: Dublett von Dublett;
m: Multiplett.

**Vergleichsbeispiel 1**

Duchführung des Verfahrens, wie in Beispiel 1 beschrieben, jedoch in Gegenwart eines gewöhnlichen, handelsüblichen Platinkatalysators [Typ Pt/C, F 101 P (DEGUSSA)] anstelle des gemäß Beispiel 1 angewandten Platinschalenkatalysators.

Das 6-Chlorchinoxalon wurde mit einem Reingehalt von 55 % in einer Ausbeute von 51,3 % der Theorie gewonnen.

Schmelzpunkt: 295-297°C.

Das $^1$H-NMR zeigt neben der Zielverbindung das Vorliegen erheblicher Mengen 6-Chlor-3,4-dihydrochinoxalon (ca. 25 Molprozent) von geringeren Mengen Chinoxalon und einer weiteren Nebenbkomponente an.

Nach HPLC (rel. Flächenprozent) enthält das Produkt 11 % 6-Chlor-3,4-dihydrochinoxalon, 3,5 % Chinoxalon und ca. 2 % 6-Chlor-2,3-dihydroxychinoxalin.

Bei Anwesenheit größerer Mengen 6-Chlor-2,3-dihydroxychinoxalin (bzw. 6-Chlorchinoxalon-N-oxid) geht die Farbe des Produkts in ein bräunliches Lila über.

**Vergleichsbeispiel 2** (entspricht Beispiel 3 der US-PS 4 636 562)

12 g 6-chlor-2-hydroxychinoxalin-4-oxid (Herstellung analog Beispiel 2 der US-PS 2 636 562) eines Reingehalts von 91,5 % (Rest: Acetat, anorganische Salze) ≙ 11,0 g (55,8 mMol) 6-Chlor-2-hydroxychinoxalin-4-oxid, gerechnet 100 %, wurden in 1200 ml 5 %iger Natronlauge bei Raumtemperatur gelöst. Nach Filtrieren der Lösung wurden ca. 16 g Raney-Nickel zugesetzt. Anschließend unter gutem Rühren Wasserstoff durchgeleitet. Nach 1 Stunde wurde mit Stickstoff gespült, der Katalysator abgetrennt und das Filtrat mit 2 N-Salzsäure angesäuert. Der Niederschlag wurde abgesaugt und im Vakuum getrocknet.

Gewonnen wurden 6,0 g 6-Chlor-2-hydroxychinoxalin vom Reingehalt 57,3 % ≙ 3,4 g (19,0 mMol) 6-Chlor-2-hydroxychinoxalin, gerechnet 100 %.

Schmelzpunkt: 287 bis 295°C. Farbe: Altrosa

Nach HPLC (rel. Flächenprozent) enthält das Produkt neben der Zielverbindung 10,4 % 2-Hydroxychinoxalin, 6,2 % 6-Chlor-3,4-dihydrochinoxalin und Spuren weiterer Nebenkomponenten.

## Patentansprüche

1. Verfahren zur Herstellung von Chinoxalonen der allgemeinen Formel (I)

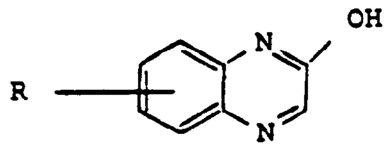

(I)

in welcher R ein Wasserstoffatom oder 1 bis 4 gleiche oder verschiedene Substituenten aus der Reihe Halogenatome, vorzugsweise Fluor- oder Chloratome, Alkyl-$C_1$-$C_4$-, Cycloalkyl-$C_3$-$C_6$-, Alkoxy-$C_1$-$C_4$-, Trifluormethyl-, Tetrafluorethoxy-, Hydroxyl-, Carboxyl-, Amino-, Monoalkyl-$C_1$-$C_4$-amino- oder Dialkyl-$C_1$-$C_4$-aminogruppen bedeuten, dadurch gekennzeichnet, daß man Chinoxalon-N-oxide der allgemeinen Formel (II)

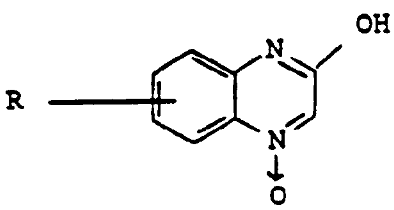

(II)

in welcher R die vorstehend genannten Bedeutungen hat und außerdem eine Nitrogruppe sein kann, die während des Verfahrens zu einer Aminogruppe reduziert wird, in wäßrig-alkalischer Lösung in Gegenwart von etwa 0,001 bis etwa 0,5 Molprozent eines Platin-Schalenkatalysators, bezogen auf eingesetztes Chinoxalon-N-oxid, bei Temperaturen von etwa 20 bis etwa 120°C bei einem Wasserstoffdruck von etwa 1 bis etwa 100 bar, in Gegenwart von etwa 1 bis etwa 25 Gewichtsprozent eines Alkalimetallhydroxids, bezogen auf die Lösung, hydriert.

2. Verfahren nach Anspruch 1, dadurch gekennzeichnet, daß man in Gegenwart von etwa 0,01 bis etwa 0,05 Molprozent des Platin-Schalenkatalysators, bezogen auf eingesetztes Chinoxalon-N-oxid, hydriert.

3. Verfahren nach mindestens einem der Ansprüche 1 und 2, dadurch gekennzeichnet, daß man bei Temperaturen von etwa 60 bis etwa 100°C hydriert.

4. Verfahren nach mindestens einem der Ansprüche 1 bis 3, dadurch gekennzeichnet, daß man bei etwa 5 bis etwa 20 bar hydriert.

5. Verfahren nach mindestens einem der Ansprüche 1 bis 4, dadurch gekennzeichnet, daß man in Gegenwart von etwa 2 bis etwa 5 Gewichtsprozent eines Alkalimetallhydroxids hydriert.

6. Verfahren nach mindestens einem der Ansprüche 1 bis 5, dadurch gekennzeichnet, daß man in Gegenwart von Natrium- oder Kaliumhydroxide hydriert.

## Claims

1. A process for the preparation of a quinoxalone of the general formula (I)

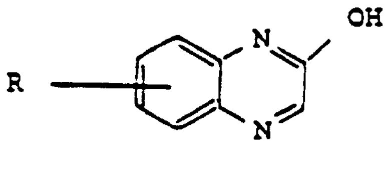

(I)

in which R denotes a hydrogen atom or 1 to 4 identical or different substituents from the series comprising halogen atoms, preferably fluorine or chlorine atom, and alkyl-$C_1$-$C_4$-, cycloalkyl-$C_3$-$C_6$-, alkoxy-$C_1$-$C_4$-, trifluoromethyl, tetrafluoroethoxy, hydroxyl, carboxyl, amino, monoalkyl-$C_1$-$C_4$-amino or dialkyl-$C_1$-$C_4$-amino groups, which comprises hydrogenating a quinoxalone N-oxide of the general formula (II)

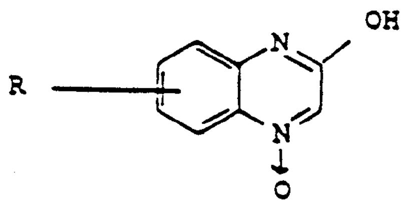

(II)

in which R has the abovementioned meanings and can also be a nitro group which is reduced to an amino group during the process, in aqueous-alkaline solution in the presence of about 0.001 to about 0.5 mol per cent of a platinum shell catalyst, based on the quinoxalone N-oxide employed, at temperatures of about 20 to about 120°C under a hydrogen pressure of about 1 to about 100 bar in the presence of about 1 to about 25 per cent by weight of an alkali metal hydroxide, based on the solution.

2. The process as claimed in claim 1, wherein the hydrogenation is carried out in the presence of about 0.01 to about 0.05 mol per cent of the platinum shell catalyst, based on the quinoxalone N-oxide employed.

3. The process as claimed in at least one of claims 1 and 2, wherein the hydrogenation is carried out at temperatures from about 60 to about 100°C.

4. The process as claimed in at least one of claims 1 to 3, wherein the hydrogenation is carried out under about 5 to about 20 bar.

5. The process as claimed in at least one of claims 1 to 4, wherein the hydrogenation is carried out in the presence of about 2 to about 5 percent by weight of an alkali metal hydroxide.

6. The process as claimed in at least one of claims 15, wherein the hydrogenation is carried out in the presence of sodium hydroxide or potassium hydroxide.

## Revendications

1. Procédé pour préparer des quinoxalones répondant à la formule générale (I) :

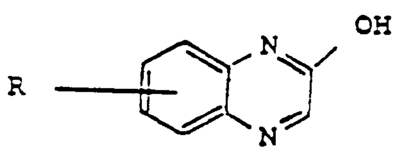

(I)

dans laquelle R représente un atome d'hydrogène ou représente de 1 à 4 substituants, identiques ou différents, pris dans l'ensemble constitué par les atomes d'halogène, de préférence les atomes de fluor et de chlore, et les radicaux alkyles en $C_1$-$C_4$, cycloalkyles en $C_3$-$C_6$, alcoxy en $C_1$-$C_4$, trifluorométhyle, tétrafluoréthoxy, hydroxy, carboxy, amino, monoalkyl-$C_1$-$C_4$-amino et dialkyl-$C_1$-$C_4$-amino, procédé caractérisé en ce qu'on hydrogène des quinoxaloneN-oxydes répondant à la formule générale (II) :

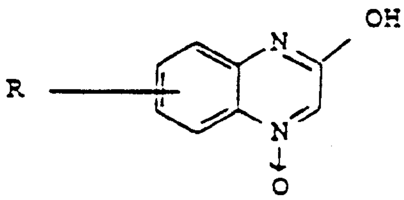

(II)

dans laquelle R a les significations qui lui ont été données ci-dessus et peut en outre représenter un radical nitro, qui est réduit en un radical amino au cours du procédé, en solution aqueuse alcaline, en présence d'environ 0,001 à environ 0,5 % en moles d'un catalyseur à coquille de platine, par rapport au quinoxalone-N-oxyde mis en jeu, à des températures d'environ 20 à environ 120°C, sous une pression d'hydrogène d'environ 1 à environ 100 bar, en présence d'environ 1 à environ 25 % en poids d'un hydroxyde de métal alcalin, par rapport à la solution.

2. Procédé selon la revendication 1 caractérisé en ce qu'on hydrogène en présence d'environ 0,01 à environ 0,05 % en moles du catalyseur à coquille de platine par rapport au quinoxalone-N-oxyde mis en jeu.

3. Procédé selon au moins une des revendications 1 et 2, caractérisé en ce qu'on hydrogène à des températures d'environ 60 à environ 100°C.

4. Procédé selon au moins une des revendications 1 à 3, caractérisé en ce qu'on hydrogène sous une pression comprise entre environ 5 et environ 20 bar.

5. Procédé selon au moins une des revendications 1 à 4, caractérisé en ce qu'on hydrogène en présence d'environ 2 à environ 5 % en poids d'un hydroxyde de métal alcalin.

6. Procédé selon au moins une des revendications 1 à 5, caractérisé en ce qu'on hydrogène en présence d'hydroxyde de sodium ou d'hydroxyde de potassium.